# EUROPEAN PATENT APPLICATION

(11) **EP 3 770 666 A1**
(43) Date of publication of application: **27.01.2021**
(21) Application number: 18910860.8
(22) Date of filing: 30.10.2018
(51) Int. Cl.: G02B 21/36, C12M 1/34, G02B 7/28, G06T 7/00

(54) **DEVICE, METHOD, AND PROGRAM FOR MEASURING DEFOCUS AMOUNT, AND DISCRIMINATOR**

(30) Priority: 22.03.2018 JP 2018053952
(71) Applicant: Fujifilm Corporation, Minato-ku Tokyo 106-8620 (JP)
(72) Inventor: WAKUI, Takashi, Ashigarakami-gun, Kanagawa 258-0023 (JP)
(74) Representative: Klunker IP Patentanwälte PartG mbB
(86) International application number: PCT/JP2018/040388
(87) International publication number: WO 2019/181053

(57) **Abstract**

A marker image detection section detects a marker image from a captured image for determining a defocus amount. A discriminator discriminates the defocus amount of the marker image included in the captured image. The discriminator performs learning using feature amounts related to a plurality of teacher marker images captured with various defocus amounts and discriminates a defocus amount of an input marker image.

## Description

### BACKGROUND OF THE INVENTION

### Technical Field

The present disclosure relates to a defocus amount measuring device, a defocus amount measuring method, a defocus amount measuring program for measuring a defocus amount of an observation target in a case where the observation target is imaged, and a discriminator that discriminates the defocus amount.

### Related Art

In the related art, a method for capturing an image of a multipotential stem cell such as an embryonic stem (ES) cell or an induced pluripotent stem (iPS) cell, a differentiated and induced cell using a microscope or the like, and capturing a feature of the image to decide a differentiation state of the cell, or the like has been proposed. Here, it has attracted attention that the multipotential stem cell such as an ES cell or an iPS cell is able to be differentiated into cells of various tissues and may be applied to regenerative medicine, development of medicines, explanation of diseases, or the like.

On the other hand, as described above, in a case where cells are imaged with a microscope, a technique for performing so-called tiling imaging has been proposed in order to acquire a high-magnification wide view image. Specifically, for example, a range of a culture container such as a well plate is scanned by an imaging optical system, and an image at each observation position is captured, and then, the images at the respective observation positions are combined. In a case where such tiling imaging is performed, a technique for acquiring a high-quality image with less blur by performing an autofocus control at each observation position in the culture container has been proposed (for example, see JP2010-072017A).

Here, as described above, in a case where the autofocus control is performed in the above-mentioned tiling imaging, it is important to perform the autofocus control at high speed and with high accuracy from the viewpoint of reducing an imaging time. However, for example, in a case where a well plate having a plurality of wells is used as a culture container, the entire well plate is scanned by an imaging optical system, and the tiling imaging is performed while performing the autofocus control for each observation position, the thickness of a bottom portion of each well varies from well to well due to a manufacturing error, or the like.

Accordingly, for example, in a case where the autofocus control is performed by detecting a position of a bottom surface (an observation target installation surface) of the well, in a case where the thickness of the bottom portion differs greatly between adjacent wells, since the position of the bottom surface of the well differs greatly, there is a problem that a time for the autofocus control becomes longer, and thus, the imaging time becomes longer. In order to solve such a problem, it is important to acquire a defocus amount in performing the autofocus control.

For this reason, various techniques for acquiring the defocus amount have been proposed. For example, in JP2013-254108A, there has been proposed a technique for fixing a sample that is an imaging target by a light transmitting member having a mark that gives at least one of a phase change or an amplitude change to transmitted light, acquiring a captured image in which an image of the sample and an image of the mark are mixed, dividing the captured image into a plurality of regions each of which includes the image of the mark, calculating an average value of the image of the mark included in each divided region as a first average value, calculating an average value of the image in each divided region as a second average value, dividing each first average value by the second average value of a corresponding region, calculating an evaluation value by averaging the values acquired through the division between regions including the same mark among the plurality of regions, and estimating a defocus amount on the basis of an evaluation value calculated for the captured image and an evaluation value calculated for a standard image that is a standard of estimation of the defocus amount.

However, since the amount of calculation for calculating the defocus amount is large in the technique disclosed in JP2013-254108A, it takes a long time to calculate the defocus amount.

### SUMMARY OF THE INVENTION

The present disclosure has been made in consideration of the above circumstances, and an object thereof is to provide a technique capable of acquiring a defocus amount at high speed.

According to an aspect of the present disclosure, there is provided a defocus amount measuring device comprising: a marker image detection section that detects a marker image from a captured image acquired by imaging a marker that is a measurement target of a defocus amount; and a discriminator that performs learning using feature amounts related to a plurality of teacher marker images captured with various defocus amounts and discriminates a defocus amount of an input marker image.

In the defocus amount measuring device according to the aspect of the present disclosure, the discriminator may discriminate the defocus amount for each of a plurality of the marker images included in the captured image, and the defocus amount measuring device may further comprise: a defocus amount determination section that determines a statistical value of a plurality of the defocus amounts as the defocus amount of the captured image.

Further, in the defocus amount measuring device according to the aspect of the present disclosure, the discriminator may discriminate that the defocus amount is not clear.

Further, in the defocus amount measuring device according to the aspect of the present disclosure, the discriminator may be configured by a neural network.

Further, in the defocus amount measuring device according to the aspect of the present disclosure, the discriminator may learn a co-occurrence matrix related to the plurality of teacher marker images as the feature amount.

Further, in the defocus amount measuring device according to the aspect of the present disclosure, the marker may have a fine cell structure.

Further, in the defocus amount measurement device according to the aspect of the present disclosure, the captured image may include the marker and may be acquired by imaging a container in which an observation target is contained, by an imaging unit, and the defocus amount measuring device may further comprise: a controller that performs a control for focusing an image of the observation target in the container on the imaging unit on the basis of the defocus amount.

The "container" may have any shape as long as it can contain an observation target. For example, a container that has a shape having a bottom portion and a continuous wall portion to the bottom portion, such as a petri dish, a dish, a flask or a well plate, may be used. Further, as the container, a micro flow channel device or the like in which a fine flow channel is formed in a plate member may be used. In addition, a container having a plate-like shape, such as a slide glass, may be used.

Further, in the defocus amount measuring device according to the aspect of the present disclosure, a stage on which the container in which the observation target is contained is placed may be further comprised, the captured image may be acquired by scanning an observation region in the container placed on the stage and performing imaging of each observation region in the container, and the controller may perform the control for focusing the image of the observation target in the container on the imaging unit on the basis of the defocus amount, in each observation region.

According to another aspect of the present disclosure, there is provided a defocus amount measuring method comprising: detecting a marker image from a captured image acquired by imaging a marker that is a measurement target of a defocus amount; and discriminating, using a discriminator that performs learning using feature amounts related to a plurality of teacher marker images captured with various defocus amounts and that discriminates a defocus amount of an input marker image, the defocus amount of the input marker image.

According to still another aspect of the present disclosure, there may be provided a program for causing a computer to execute the defocus amount measuring method.

According to still another aspect of the present disclosure, there is provided a defocus amount measuring device comprising a memory that stores a command to be executed by a computer; and a processor configured to execute the stored command, in which the processor executes: a process of detecting a marker image from a captured image acquired by imaging a marker that is a measurement target of a defocus amount; and a process of discriminating, using a discriminator that performs learning using feature amounts related to a plurality of teacher marker images captured with various defocus amounts and that discriminates a defocus amount of an input marker image, the defocus amount of the input marker image.

According to still another aspect of the present disclosure, there is provided a discriminator that performs learning using feature amounts related to a plurality of teacher marker images captured with various defocus amounts and discriminates a defocus amount of an input marker image.

According to still another aspect of the present disclosure, there is provided a defocus amount measuring device comprising a discriminator that performs learning using feature amounts related to a plurality of teacher marker images captured with various defocus amounts and discriminates, in a case where a captured image acquired by imaging a marker that is a measurement target of a defocus amount is input, a presence or absence of a marker image in the captured image and a defocus amount of the marker image in a case where the marker image is included in the captured image.

According to still another aspect of the present disclosure, there is provided a discriminator that performs learning using feature amounts related to a plurality of teacher marker images captured with various defocus amounts and in a case where a captured image acquired by imaging a marker that is a measurement target of a defocus amount is input, discriminates a presence or absence of a marker image in the captured image and a defocus amount of the marker image in a case where the marker image is included in the captured image.

According to the present disclosure, a marker image is detected from a captured image including a marker that is a measurement target of a defocus amount, learning is performed by using feature amounts related to a plurality of teacher marker images captured with various defocus amounts, and the defocus amount is discriminated by a discriminator that discriminates a defocus amount of an input marker image. Accordingly, it is possible to determine the defocus amount at high speed with a small amount of calculation.

### BRIEF DESCRIPTION OF THE DRAWINGS

Fig. 1 is a block diagram showing a schematic configuration of a microscope device in a microscope imaging system to which a defocus amount measuring device according to a first embodiment is applied.
Fig. 2 is a schematic diagram showing a configuration of an imaging optical system.
Fig. 3 is a perspective view showing a configuration of a stage.
Fig. 4 is a schematic diagram showing a configuration of a focal length changing optical system.
Fig. 5 is a block diagram showing a schematic configuration of a microscope observation system that uses the defocus amount measuring device according to the first embodiment of the present disclosure.
Fig. 6 is a diagram for illustrating imaging of a marker for acquiring a teacher marker image to be used for learning of a discriminator.
Fig. 7 is a diagram showing an example of the teacher marker image.
Fig. 8 is a diagram showing a discrimination result of a defocus amount.
Fig. 9 is a diagram showing a scanning position of an observation region in a culture container.
Fig. 10 is a flowchart showing a process performed in the first embodiment.
Fig. 11 is a flowchart showing a process performed in a second embodiment.
Fig. 12 is a diagram for illustrating an autofocus control.
Fig. 13 is a block diagram showing a schematic configuration of a microscope observation system that uses a defocus amount measuring device according to a third embodiment of the present disclosure.

### DETAILED DESCRIPTION

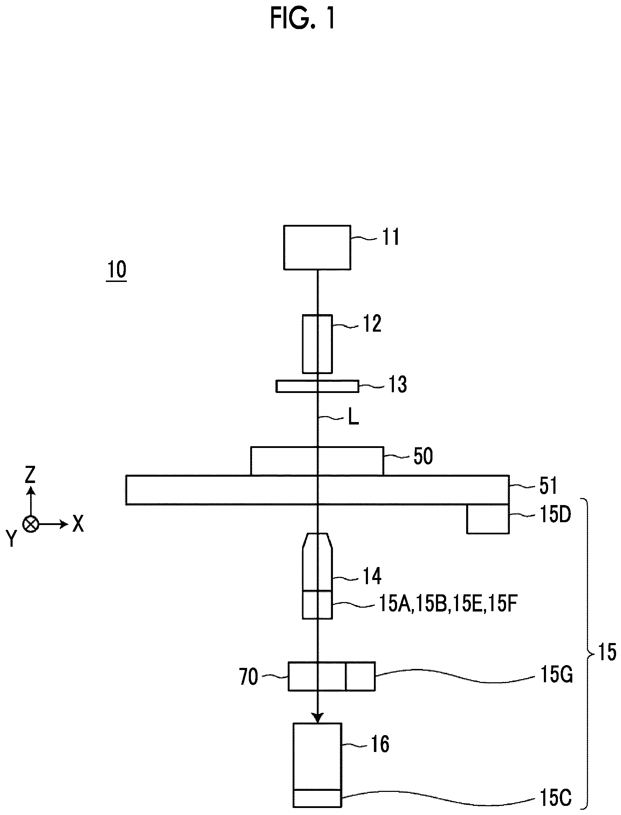

Hereinafter, a microscope imaging system to which a defocus amount measuring device, a defocus amount measuring method, and a defocus amount measuring program according to an embodiment of the present disclosure are applied will be described in detail with reference to the accompanying drawings. Fig. 1 is a block diagram showing a schematic configuration of a microscope device in a microscope imaging system to which a defocus amount measuring device according to a first embodiment of the present disclosure is applied.

A microscope device 10 performs imaging for acquiring a phase difference image of a cultured cell that is an observation target. Specifically, as shown in Fig. 1, the microscope device 10 comprises a white light source 11 that emits white light, a condenser lens 12, a slit plate 13, an imaging optical system 14, an operation section 15, and an imaging unit 16. Further, the microscope device 10 comprises a focal length changing optical system 70.

The operation section 15 comprises a first operation section 15A, a second operation section 15B, a third operation section 15C, a fourth operation section 15D, a fifth operation section 15E, a sixth operation section 15F, and a seventh operation section 15G. Operations of the first to seventh operation sections 15A to 15G will be described later.

The slit plate 13 has a configuration in which a light screen that screens white light emitted from the white light source 11 is formed with a ring-shaped slit through which the white light passes, in which ring-shaped illumination light L is formed as the white light passes through the slit.

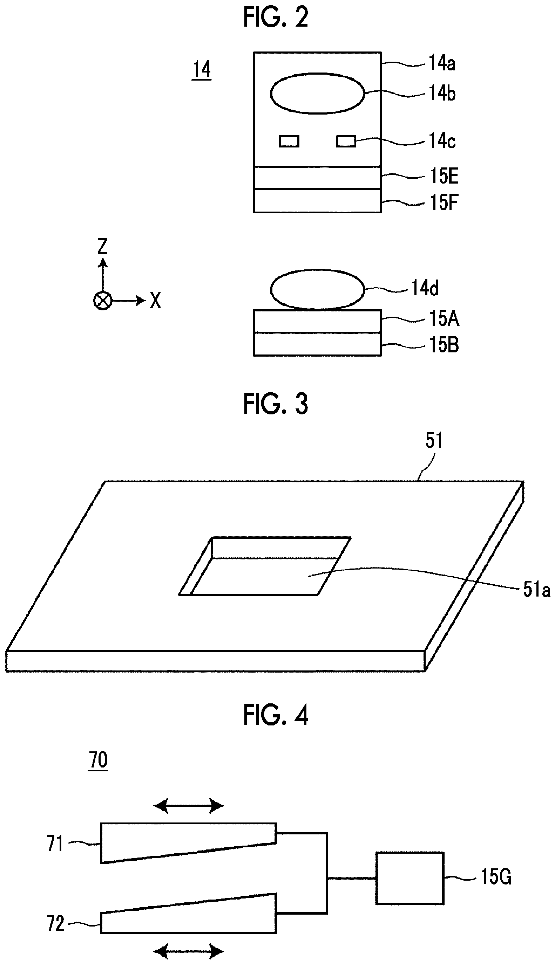

The imaging optical system 14 forms a phase difference image for each observation region obtained by dividing the inside of a range of the culture container 50 on the imaging unit 16. Fig. 2 is a diagram showing a detailed configuration of the imaging optical system 14. The imaging optical system 14 comprises a phase difference lens 14a and an imaging lens 14d, as shown in Fig. 2. Further, the phase difference lens 14a comprises an objective lens 14b and a phase plate 14c. The phase plate 14c has a configuration in which a phase ring is formed in a transparent plate that is transparent with respect to a wavelength of the illumination light L. The size of the slit of the above-described slit plate 13 has a conjugate relationship with the phase ring of the phase plate 14c.

The phase ring has a configuration in which a phase membrane that shifts a phase of incident light by 1/4 of a wavelength and a dimmer filter that dims incident light are formed in a ring shape. The phase of direct light incident onto the phase ring shifts by 1/4 of a wavelength after passing through the phase ring, and its brightness is weakened. On the other hand, most of diffracted light diffracted by an observation target passes through the transparent plate of the phase plate 14c, and its phase and brightness are not changed.

The phase difference lens 14a having the objective lens 14b is moved in an optical axis direction of the objective lens 14b by the fifth operation section 15E of the operation section 15 shown in Fig. 1. In this embodiment, the optical axis direction of the objective lens 14b and a Z direction (vertical direction) are the same direction. As the objective lens 14b is moved in the Z direction, an autofocus control is performed, and contrast of a phase difference image acquired by the imaging unit 16 is adjusted.

Further, a configuration in which a magnification of the phase difference lens 14a is changeable may be used. Specifically, a configuration in which the phase difference lenses 14a or the imaging optical system 14 having different magnifications are exchangeable may be used. The exchange or the phase difference lens 14a or the imaging optical system 14 may be automatically performed, or may be manually performed by a user.

Further, the objective lens 14b consists of a liquid lens whose focal length is changeable. As long as the focal length can be changed, the objective lens 14b is not limited to the liquid lens, and any other lens such as a liquid crystal lens or a shape deformable lens may be used. In the objective lens 14b, an applied voltage is changed by the sixth operation section 15F in the operation section 15 shown in Fig. 1, so that the focal length is changed. Thus, the focal length of the imaging optical system 14 is changed. Due to the change of the focal length of the objective lens 14b, similarly, the autofocus control is performed, and the contrast of the phase difference image acquired by the imaging unit 16 is adjusted.

The imaging lens 14d receives a phase difference image passed through the phase difference lens 14a, which is incident thereonto, and causes the image to be formed on the imaging unit 16. In this embodiment, the imaging lens 14d consists of a liquid lens whose focal length is changeable. As long as the focal length can be changed, the objective lens 14b is not limited to the liquid lens, and any other lens such as a liquid crystal lens or a shape deformable lens may be used. In the imaging lens 14d, an applied voltage is changed by the first operation section 15A in the operation section 15 shown in Fig. 1, so that the focal length is changed. Thus, the focal length of the imaging optical system 14 is changed. Due to the change of the focal length of the imaging lens 14d, similarly, the autofocus control is performed, and the contrast of the phase difference image acquired by the imaging unit 16 is adjusted.

The imaging lens 14d is moved in the optical axis direction of the imaging lens 14d by the second operation section 15B in the operation section 15 shown in Fig. 1. In this embodiment, the optical axis direction of the imaging lens 14d and the Z direction (vertical direction) are the same direction. As the imaging lens 14d is moved in the Z direction, the autofocus control is performed, and the contrast of the phase difference image acquired by the imaging unit 16 is adjusted.

The imaging unit 16 acquires a phase difference image formed by the imaging lens 14d. As the imaging unit 16, an imaging element such as a charge-coupled device (CCD) image sensor, a complementary metal-oxide semiconductor (CMOS) image sensor may be comprised. As the imaging element, an imaging element in which color filters of red, green, and blue (R, G, and B) are provided may be used, or a monochromic imaging element may be used.

Further, the imaging unit 16 is moved in the Z direction by the third operation section 15C in the operation section 15 shown in Fig. 1. In this embodiment, a direction perpendicular to an imaging surface of the imaging unit 16 and the Z direction are the same direction. As the imaging unit 16 is moved in the Z direction, the autofocus control is performed, and the contrast of the phase difference image acquired by the imaging unit 16 is adj usted.

A stage 51 is provided between the slit plate 13 and the imaging optical system 14. A culture container 50 in which cells that are observation targets are contained is disposed on the stage 51.

The culture container 50 corresponds to a container of the present disclosure. As the culture container 50, a petri dish, a dish, a flask, a well plate, or the like may be used. In addition, as the container, a slide glass, a micro flow channel device in which fine flow channels are processed, or the like, may be used. In addition, as cells contained in the culture container 50, multipotential stem cells such as iPS cells and ES cells, cells of nerves, the skin, the myocardium and the liver, which are differentiated and induced from a stem cell, cells of the skin, the retina, the myocardium, blood corpuscles, nerves, and organs extracted from a human body may be used.

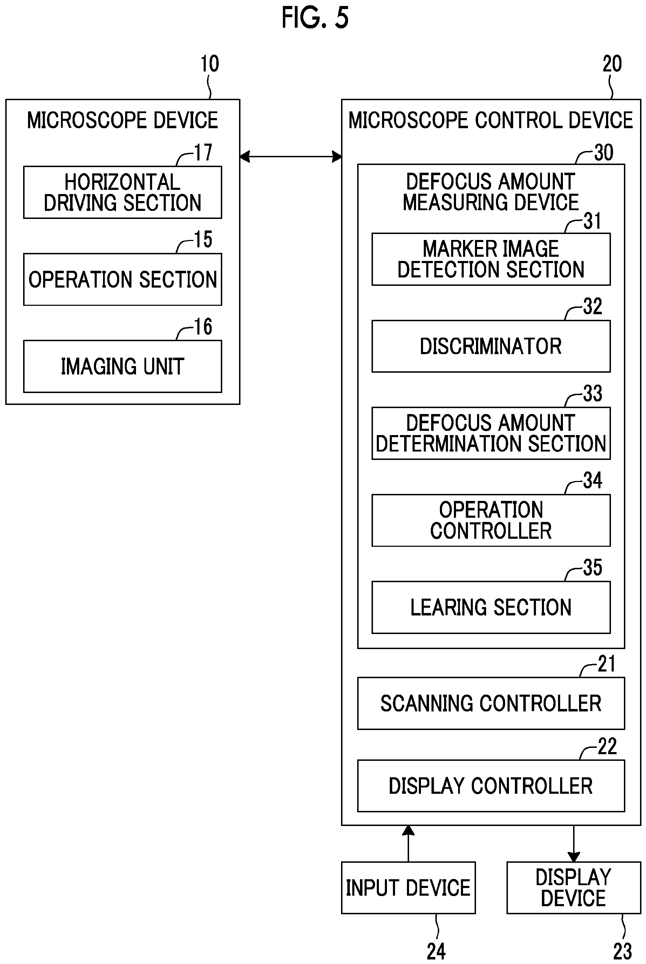

The stage 51 is configured to be moved in an X direction and a Y direction that are orthogonal to each other by a horizontal driving section 17 to be described later (see Fig. 5). The X direction and the Y direction are directions that are orthogonal to a Z direction, and are directions that are orthogonal to each other in a horizontal plane. In the present embodiment, the X direction is a main scanning direction, and the Y direction is a sub scanning direction.

Fig. 3 is a diagram showing an example of the stage 51. At the center of the stage 51, a rectangular opening 51a is formed. The culture container 50 is provided on a member that is formed with the opening 51a, and in this configuration, a phase difference image of a cell in the culture container 50 passes through the opening 51a.

Further, the stage 51 is moved in the Z direction by the fourth operation section 15D, and thus, the culture container 50 is moved in the Z direction. The fourth operation section 15D comprises an actuator such as a piezoelectric element, for example. In the present embodiment, a direction perpendicular to a surface of the stage 51 on which the culture container 50 is provided and the Z direction are the same direction. As the stage 51 is moved in the Z direction, similarly, the autofocus control is performed, and the contrast of the phase difference image acquired by the imaging unit 16 is adjusted.

The first operation section 15A and the sixth operation section 15F are provided with, for example, a voltage variable circuit. The first operation section 15A changes a voltage to be applied to the imaging lens 14d on the basis of a control signal output from the defocus amount measuring device 30 to be described later. The sixth operation section 15F changes a voltage to be applied to the objective lens 14b on the basis of a control signal output from the defocus amount measuring device 30 to be described later.

The second operation section 15B, the third operation section 15C, the fourth operation section 15D, and the fifth operation section 15E are provided with actuators such as piezoelectric elements, and are driven on the basis of control signals output from the defocus amount measuring device 30 to be described later. The operation section 15 is configured to pass the phase difference image that has passed through the phase difference lens 14a and the imaging lens 14d as it is. The configurations of the second operation section 15B, the third operation section 15C, the fourth operation section 15D, and the fifth operation section 15E are not limited to the piezoelectric element, and as long as the imaging lens 14d, the imaging unit 16, the stage 51, and the objective lens 14b (phase difference lens 14a) can be moved in the Z direction, and any other known configuration may be used.

Fig. 4 is a schematic diagram showing a configuration of the focal length changing optical system. As shown in Fig. 4, the focal length changing optical system 70 comprises a circular first wedge prism 71 and a circular second wedge prism 72. The seventh operation section 15G moves the first wedge prism 71 and the second wedge prism 72 to be synchronized with each other in opposite directions. With this configuration, a focal position of the imaging optical system 14 is changed. The change of the focal position means that the focal length increases or decreases. Thus, as the focal position of the imaging optical system 14 is changed, the focal length of the imaging optical system 14 is changed. In the present embodiment, the change of the focal length of the imaging optical system 14 includes the change of the focal length of the imaging lens 14d by the first operation section 15A, and the change of the focal length of the objective lens 14b by the sixth operation section 15F, and additionally, the change of the focal length of the imaging optical system 14 due to the change of the focal position of the imaging optical system 14 by the seventh operation section 15G.

The first and second wedge prisms 71 and 72 are prisms in which two surfaces that can be a light incident surface and a light emitting surface are not parallel, that is, one surface is inclined with respect to the other surface. In the following description, a surface arranged perpendicular to the optical axis is referred to as a right-angled surface, and a surface arranged inclined with respect to the optical axis is referred to as a wedge surface. The wedge prisms 71 and 72 are prisms that deflect light that is incident perpendicularly to the right-angled surface. The seventh operation section 15G comprises an actuator such as a piezoelectric element, for example, and moves the first wedge prism 71 and the second wedge prism 72 to be synchronized with each other in opposite directions while maintaining the right-angled surfaces in parallel on the basis of control signals output from the defocus amount measuring device 30 to be described later. That is, in a case where the first wedge prism 71 is moved rightward in Fig. 4, the second wedge prism 72 is moved leftward. Conversely, in a case where the first wedge prism 71 is moved leftward in Fig. 4, the second wedge prism 72 is moved rightward. By moving the first and second wedge prisms 71 and 72 in this way, an optical path length of light emitted from the imaging optical system 14 is changed, so that the focal position of the imaging optical system 14 is changed, to thereby make it possible to change the focal length. Accordingly, the autofocus control is performed, and the contrast of the phase difference image acquired by the imaging unit 16 is adjusted.

Next, a configuration of the microscope control device 20 that controls the microscope device 10 will be described. Fig. 5 is a block diagram showing a configuration of the microscope observation system according to the first embodiment. With respect to the microscope device 10, a block diagram of a partial configuration controlled by respective sections of the microscope control device 20 is shown.

The microscope control device 20 generally controls the microscope device 10, and comprises the defocus amount measuring device 30, the scanning controller 21, and the display controller 22 according to the first embodiment. Further, the defocus amount measuring device 30 comprises a marker image detection section 31, a discriminator 32, a defocus amount determination section 33, an operation controller 34, and a learning section 35 for the discriminator 32. The operation controller 34 corresponds to a controller of the present disclosure.

The microscope control device 20 is configured of a computer comprising a central processing unit, a semiconductor memory, a hard disk, and the like, and an embodiment of a defocus amount measuring program of the present disclosure and a microscope control program are installed in the hard disk. Here, as the defocus amount measuring program and the microscope control program are executed by the central processing unit, the marker image detection section 31, the discriminator 32, the defocus amount determination section 33, the operation controller 34, and the learning section 35, the scanning controller 21, and the display controller 22 shown in Fig. 5 perform their functions.

Here, in the present embodiment, a marker is included in a culture container 50 in order to measure the defocus amount for performing the autofocus control. As the marker, for example, a pattern at the time of processing formed on the surface of the culture container 50, fine beads put into the culture container 50, a fine structure of cells contained in the culture container 50 (for example, nucleoli), or the like may be used. Here, the culture container 50 is manufactured by injection molding of a resin material, and the surface thereof has a pattern formed on the surface of a mold during cutting of the mold. The pattern formed on the surface of the culture container 50 may be used as a marker. Further, the fine beads are made of resin spheres such as polyester having a diameter of 1 to 2 µm. Such fine beads may be put into the culture container 50, and may be used as a marker. Further, since the fine structure of cells such as nucleoli is spherical, such fine structure of cells may be used as a marker.

In the present embodiment, in order to determine the defocus amount, the imaging unit 16 acquires an image (hereinafter, referred to as a captured image G0) for determining the defocus amount prior to the acquisition of the phase difference image.

The marker image detection section 31 detects a marker image from the captured image G0 for determining the defocus amount, which is acquired by the imaging unit 16. In the present embodiment, the captured image G0 is a phase difference image, and the above-described marker is represented by a different contrast with respect to a background image in the phase difference image. Accordingly, the marker image detection section 3 1 detects the marker image from the captured image G0 by performing threshold value processing.

The discriminator 32 performs learning using feature amounts related to a plurality of teacher marker images captured by changing focus shift amounts, that is, the plurality of teacher marker images captured with various defocus amounts, and discriminates a defocus amount of the marker image input by an input of the marker image.

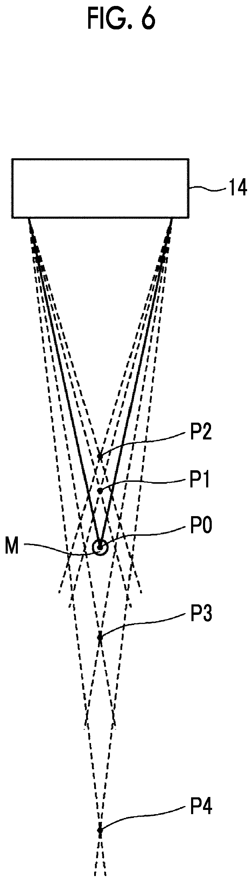

Hereinafter, the learning of the discriminator 32 will be described. The learning of the discriminator 32 is performed by the learning section 35. Fig. 6 is a diagram for illustrating imaging of a marker for acquiring a teacher marker image used for learning of the discriminator 32. Referring to Fig. 6, imaging of one marker M will be described. As shown in Fig. 6, in order to acquire a teacher marker image, a marker M is imaged at a plurality of focus positions. That is, first, the imaging optical system 14 is adjusted to perform a focus control for focusing on a position P0 of the marker M, and an image focused on the marker M is acquired. Further, the focus control is performed for focusing on a position P1 and a position P2 in front of the marker M, and images defocused in a positive direction are acquired. Further, the focus control is performed for focusing on a position P3 and a position P4 behind the marker M, and images defocused in a negative direction are acquired. In Fig. 6, the marker M is imaged at the five focus positions P0 to P4, but the present invention is not limited thereto, and the marker M may be imaged at more or less focus positions.

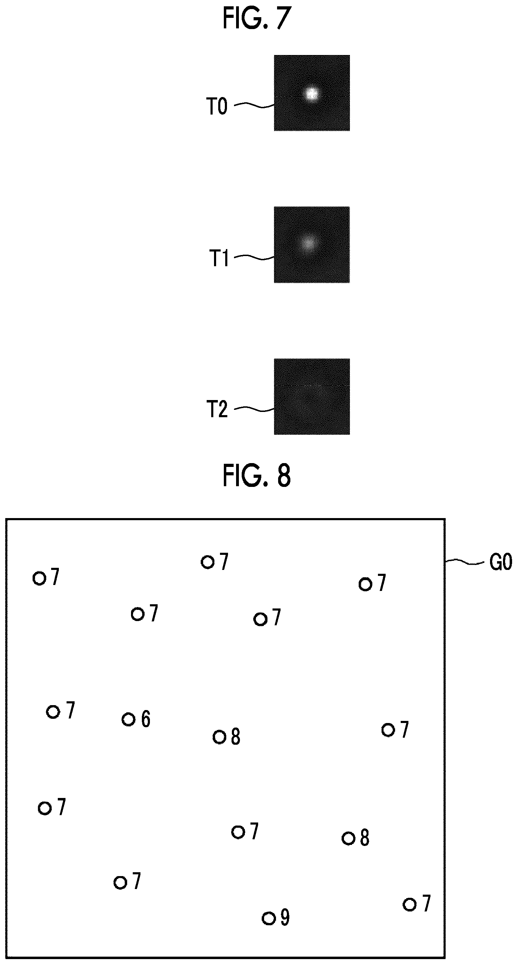

Then, the learning section 35 extracts a region including the marker from the images acquired by imaging the marker M at the plurality of focus positions as described above, and generates a teacher marker image. Fig. 7 is a diagram showing an example of teacher marker images. Fig. 7 shows teacher marker images T0, T1, and T2 generated from the images acquired by focusing on the positions P0, P1, and P2. A large number of (for example, 1000) teacher marker images are prepared at respective focus positions.

Further, the learning section 35 also associates the defocus amount with the teacher marker image. For example, the teacher marker image acquired at the focus position P0 is associated with 0 as a defocus amount, the teacher marker image acquired at the focus position P1 is associated with +6 µm as a defocus amount, and the teacher marker image acquired at the focus position P2 is associated with +12 µm as a defocus amount. Further, the teacher marker image acquired at the focus position P3 is associated with -6 µm as a defocus amount, and the teacher marker image acquired at the focus position P4 is associated with -12 µm as a defocus amount.

The learning section 35 causes the discriminator 32 to perform learning so as to discriminate the defocus amount of the input marker image using the teacher marker image. In the present embodiment, the discriminator 32 discriminates the defocus amount of the marker image in a case where the marker image that is a discrimination target is input. Specifically, the discriminator 32 calculates probabilities of a plurality of defocus amounts for the marker image that is the discrimination target, and discriminates a defocus amount having the highest probability is input as the defocus amount of the input marker image. Accordingly, the learning section 35 acquires feature amounts in a region having a predetermined size (for example, 3 × 3) from the teacher marker images, inputs the acquired feature amounts to the discriminator 32, and performs learning, that is, machine learning of the discriminator 32 to output discrimination results that become defocus amounts corresponding to the input teacher marker images.

The discriminator 32 may be configured of a support vector machine (SVM), a deep neural network (DNN), a convolutional neural network (CNN), a recurrent neural network (RNN), or the like.

Further, a co-occurrence matrix related to the teacher marker images may be used as the feature amounts of the teacher marker images. The co-occurrence matrix is a matrix that shows distribution of signal values of pixels in an image, in which the frequencies of signal values of pixels adjacent to a pixel having a certain signal value are represented as a matrix. Here, in a case where the defocus amount of the marker image is 0, that is, in a case where the marker image is in focus, since the contrast of the marker image is high, a pixel adjacent to a pixel having high brightness (that is, low density) has low brightness (that is, high density). Accordingly, in a case where the defocus amount of the marker image is 0, the frequency that a pixel having a high signal value is adjacent to the pixel having high brightness becomes high. On the other hand, in a case where the marker image is blurred, the brightness of a pixel adjacent to a pixel having high brightness pixel is not so much low. For this reason, in a case where the marker image is blurred, since the contrast of the marker image is low, the frequency that a pixel having a signal value that becomes a similar brightness is adjacent to the pixel having high brightness becomes high. For this reason, the co-occurrence matrix related to the teacher marker images becomes a characteristic matrix in accordance with the degree of blurring of the marker image. Accordingly, by using the co-occurrence matrix as the feature amounts, it is possible to cause the discriminator 32 to perform learning so that the defocus amounts can be accurately discriminated.

The discriminator 32 that has performed learning in this way discriminates the defocus amount of the marker included in the captured image G0 acquired by the imaging unit 16. Fig. 8 is a diagram showing a discrimination result of a defocus amount. In the captured image G0 shown in Fig. 8, the nucleoli of cells are used as markers, and marker images are shown by white circles in Fig. 8. The discriminator 32 discriminates the defocus amount for each of the plurality of marker images included in the captured image G0 as shown in Fig. 8. In Fig. 8, for ease of description, a numerical value (µm) that represents the defocus amount for each marker image is shown in the vicinity of each marker image.

The defocus amount determination section 33 determines a statistical value of the defocus amounts of the plurality of marker images discriminated by the discriminator 32 for one captured image G0 as the defocus amount of the captured image G0. As the statistical value, an average value, a median value, a mode value, or the like of the defocus amounts of the plurality of marker images may be used. For example, in a case where the statistical value is the mode value, the statistical value of the defocus amounts is determined to be 7 µm for the captured image G0 for which the defocus amounts are discriminated as shown in Fig. 8.

The operation controller 34 operates the operation section 15 to perform an autofocus control on the basis of the defocus amount determined by the defocus amount determination section 33 as described above. Specifically, the operation controller 34 outputs a control signal to each of the first operation section 15A to the seventh operation section 15G on the basis of the defocus amount. Thus, the focal length of the imaging lens 14d is changed by the first operation section 15A, and thus, the focal length of the imaging optical system 14 is changed. Further, the imaging lens 14d is moved in the optical axis direction by the second operation section 15B. In addition, the imaging unit 16 is moved in the optical axis direction by the third operation section 15C. Further, the stage 51 is moved in the optical axis direction by the fourth operation section 15D. In addition, the objective lens 14b is moved in the optical axis direction by the fifth operation section 15E. The focal length of the objective lens 14b is changed by the sixth operation section 15F, and thus, the focal length of the imaging optical system 14 is changed. Further, the focal position of the imaging optical system 14 is changed by the seventh operation section 15G, and thus, the focal length of the imaging optical system 14 is changed. Through these seven operations, the autofocus control is performed.

The scanning controller 21 controls driving of the horizontal driving section 17 to move the stage 51 in the X direction and the Y direction, to thereby move the culture container 50 in the X direction and the Y direction. The horizontal driving section 17 is configured by an actuator such as a piezoelectric element.

Hereinafter, the movement control of the stage 51 by the scanning controller 21 and the autofocus control by the operation controller 34 will be described in detail.

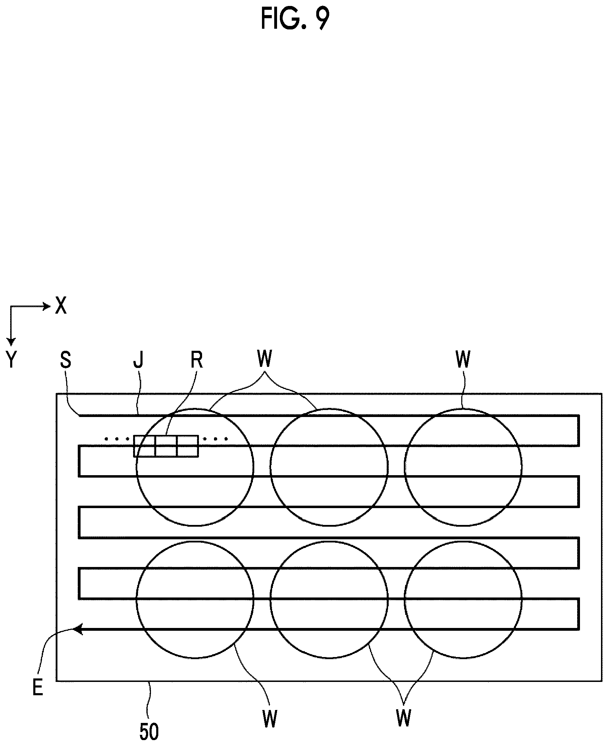

In the present embodiment, the stage 51 is moved in the X direction and the Y direction under the control of the scanning controller 21, an observation region of the imaging optical system 14 is moved two-dimensionally within the culture container 50 to scan the culture container 50, and each observation region is imaged to acquire a phase difference image. Fig. 9 is a diagram showing a scanning position according to an observation region in the culture container 50 using a solid line J. In this embodiment, a well plate having six wells W is used as the culture container 50.

As shown in Fig. 9, an observation region of the imaging optical system 14 moves from a scanning start point S to a scanning end point E along the solid line J. That is, the observation region R is moved in a positive direction (a rightward direction in Fig. 9) of the X direction, is moved in the Y direction (a downward direction in Fig. 9), and then, is moved in a reverse negative direction (in a leftward direction in Fig. 9). Then, the observation region R is moved again in the Y direction, and is moved again in the positive direction. In this way, by repeating the reciprocating movement of the observation region R in the X direction and the movement of the observation region R in the Y direction, the culture container 50 is scanned in a two-dimensional manner.

Further, in the present embodiment, the stage 51 is once stopped in each observation region R. In this state, the captured image G0 for determining the defocus amount is acquired by the imaging unit 16, the defocus amount is determined, the autofocus control is performed on the basis of the defocus amount, and the observation region R is imaged to acquire a phase difference image. After the phase difference image is acquired, the stage 51 is moved, and the autofocus control is performed in the next observation region R to acquire a phase difference image. By repeating this operation, a plurality of phase difference images that represent the entire culture container 50 are acquired, and the plurality of phase difference images are combined to generate a composite phase difference image.

That is, the operation controller 34 performs the autofocus control by controlling the driving of the operation section 15 on the basis of the defocus amount determined in the observation region R. Specifically, the operation controller 34 stores relationships between the defocus amount; a voltage applied to the imaging lens 14d, the amount of movement of the imaging lens 14d in the optical axis direction, the amount of movement of the imaging unit 16 in the optical axis direction, the amount of movement of the stage 51 in the optical axis direction, and the amount of movement of the objective lens 14b in the optical axis direction for changing the focal length of the imaging lens 14d; and a voltage applied to the objective lens 14b and the amount of movement of the focal length changing optical system 70 for changing the focal length of the objective lens 14b in advance as a table. This table is referred to as a first table.

The operation controller 34 respectively obtains the voltage applied to the imaging lens 14d, the amount of movement of the imaging lens 14d in the optical axis direction, the amount of movement of the imaging unit 16 in the optical axis direction, the amount of movement of the stage 51 in the optical axis direction, and the amount of movement of the objective lens 14b in the optical axis direction for changing the focal length of the imaging lens 14d; and the voltage applied to the objective lens 14b and the amount of movement of the focal length changing optical system 70 for changing the focal length of the objective lens 14b, with reference to the first table, on the basis of the determined defocus amount. In the following description, the voltage applied to the imaging lens 14d, the amount of movement of the imaging lens 14d in the optical axis direction, the amount of movement of the imaging unit 16 in the optical axis direction, the amount of movement of the stage 51 in the optical axis direction, and the amount of movement of the objective lens 14b in the optical axis direction for changing the focal length of the imaging lens 14d, and the voltage applied to the objective lens 14b for changing the focal length of the objective lens 14b and the amount of movement of the focal length changing optical system 70 are referred to as focus control amounts.

The operation controller 34 outputs control signals corresponding to the focus control amounts to the first operation section 15A to the seventh operation section 15G in order to control the operation section 15. Specifically, the operation controller 34 acquires the focus control amounts with reference to the first table on the basis of the defocus amount, and outputs the focus control amounts to the first operation section 15A to the seventh operation section 15G.

The operation section 15, that is, the first operation section 15A to the seventh operation section 15G are driven on the basis of the input control signals. Thus, the focus control is performed according to the defocus amount of the observation region R.

Returning to Fig. 5, the display controller 22 combines phase difference images in the respective observation regions R captured by the microscope device 10 to generate one composite phase difference image, and displays the composite phase difference image on the display device 23.

The display device 23 displays the composite phase difference image generated by the display controller 22 as described above, and comprises a liquid crystal display, or the like, for example. Further, the display device 23 may be formed by a touch panel, which may also be used as the input device 24.

The input device 24 comprises a mouse, a keyboard, and the like, and receives various setting inputs from a user. The input device 24 according to this embodiment receives setting inputs such as a change command of the magnification of the phase difference lens 14a, a change command of the moving velocity of the stage 51, for example.

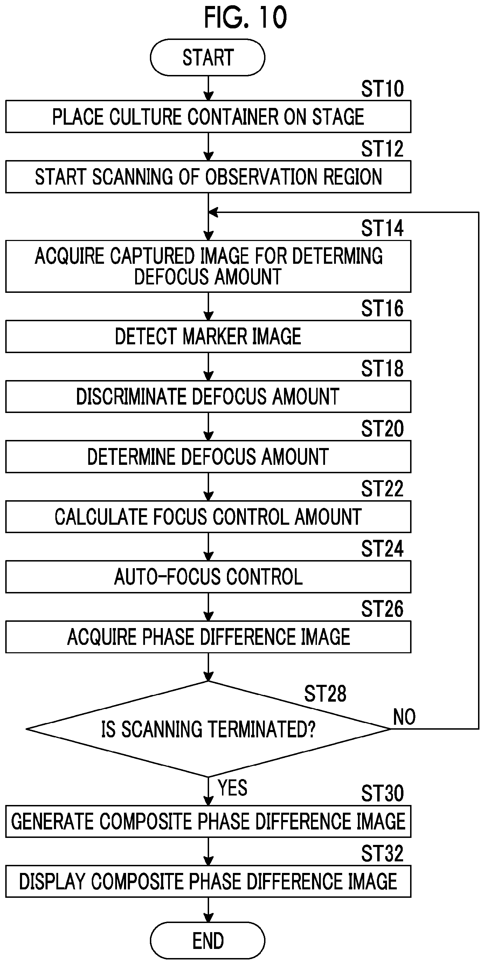

Next, an operation of the microscope observation system to which the defocus amount measuring device according to the first embodiment is applied will be described with reference to a flowchart shown in Fig. 10. First, the culture container 50 in which cells that are observation targets are contained is provided on the stage 51 (step ST10). Then, the stage 51 is moved so that the observation region R of the imaging optical system 14 is set to the position of the scanning start point S shown in Fig. 6, and scanning according to the observation region R is started (step ST12).

Here, in the present embodiment, as described above, for each observation region R, the captured image G0 for determining the defocus amount is acquired, the marker image is detected, the defocus amount is discriminated, the defocus amount is determined, the focus control amount is calculated, the autofocus control is performed, and the phase difference image is acquired. These operations are performed while moving the observation region R. That is, after the acquisition of the captured image G0, the detection of the marker image, the discrimination of the defocus amount, the determination of the defocus amount, the calculation of the focus control amount, the autofocus control, and the acquisition of the phase difference image are performed for an observation region R at a certain position, for the next observation region R, the acquisition of the captured image G0, the detection of the marker image, the discrimination of the defocus amount, the determination of the defocus amount, the calculation of the focus control amount, the autofocus control, and the acquisition of the phase difference image are performed.

Accordingly, in the first observation region R, the captured image G0 for determining the defocus amount is acquired by the imaging unit 16 (step ST14), and the marker image detection section 31 detects a marker image from the captured image G0 (step ST16). Then, the discriminator 32 discriminates the defocus amount of the marker image included in the captured image G0 (step ST18), and the defocus amount determination section 33 determines the defocus amount in the observation region R (step ST20). Then, the operation controller 34 calculates the focus control amount on the basis of the determined defocus amount (step ST22), and performs the autofocus control on the basis of the focus control amount (step ST24). That is, the operation controller 34 controls the driving of the operation section 15 on the basis of the amount of movement that is previously stored, changes the focal length of the imaging lens 14d, and moves the imaging lens 14d, the imaging unit 16, and the objective lens 14b in the Z direction. Then, after the autofocus control, the imaging unit 16 images the observation region R to acquire a phase difference image in the observation region R (step ST26). The acquired phase difference image is output from the imaging unit 16 to the display controller 22 for storage.

Then, in a case where the entire scanning is not terminated (step ST28; NO), the observation region R is moved in the X direction or the Y direction, and the acquisition of the captured image G0, the detection of the marker image, the discrimination of the defocus amount, the determination of the defocus amount, the calculation of the focus control amount, the autofocus control, and the acquisition of the phase difference image that have been described above are repeatedly performed until the entire scanning is terminated (step ST14 to step ST26). Further, at a time point when the observation region R reaches the position of the scanning end point E shown in Fig. 9, the entire scanning is terminated (step ST28; YES).

After the entire scanning is terminated, the display controller 22 combines phase difference images of the respective observation regions R to generate a composite phase difference image (step ST30), and displays the generated composite phase difference image on the display device 23 (step ST32).

As described above, in the present embodiment, the captured image G0 for determining the defocus amount, which includes the marker that is the measurement target of the defocus amount, is acquired, the marker image is detected from the captured image G0, and learning is performed using the feature amounts related to the plurality of teacher marker images captured with various defocus amounts, and the defocus amount is discriminated by the discriminator 32 that discriminates the defocus amount of the input marker image. Accordingly, it is possible to determine the defocus amount at high speed with a small amount of calculation.

Further, by discriminating the defocus amount for each of the plurality of marker images included in the captured image G0 and determining the statistical value of the plurality of defocus amounts as the defocus amount of the observation region R in which the captured image G0 is acquired, it is possible absorb variation in the discrimination results of the discriminator 32, to thereby accurately determine the defocus amount.

Further, by focusing an image of an observation target in the culture container 50 on the imaging unit 16 on the basis of the defocus amount, it is possible to determine the defocus amount at high speed, and thus, it is possible to perform the autofocus control at high speed.

In the first embodiment, the defocus amount measuring device 30 according to the first embodiment is applied to a microscope imaging system, and the acquisition of the captured image G0, the detection of the marker image, the discrimination of the defocus amount, the determination of the defocus amount, the calculation of the focus control amount, the autofocus control, and the acquisition of the phase difference image are performed in each observation region R while moving the observation region R, but the invention is not limited thereto. For example, a configuration in which, with respect to a certain culture container 50, the acquisition of the captured image G0, the detection of the marker image, the discrimination of the defocus amount, the determination of the defocus amount, and the calculation of the focus control amount are performed in each observation region R of the culture container 50 without containing cells may be used. In this case, after the defocus amounts are determined in all the observation regions R of the culture container 50, cells contained in the culture container 50 of the same type as the culture container 50 for which the defocus amount is determined are observed, and the phase difference image is acquired. In this way, in a case where the defocus amount is determined prior to the acquisition of the phase difference image, it is preferable that fine beads are used as the markers M. Hereinafter, this configuration will be described as a second embodiment.

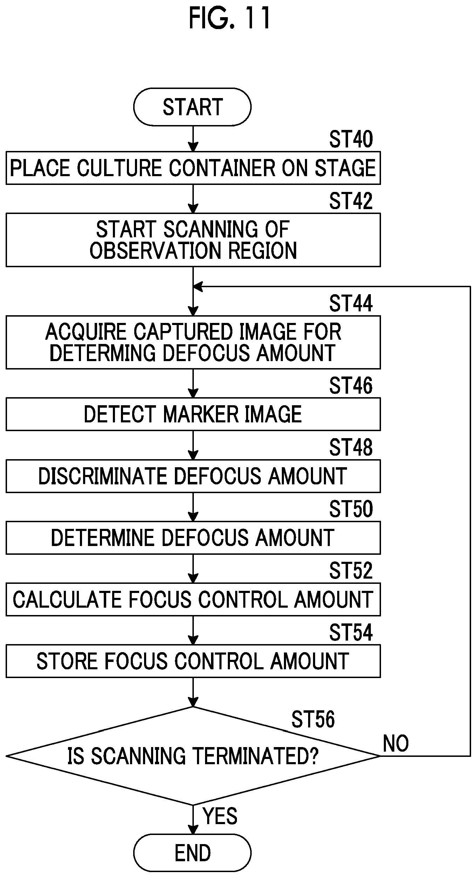

Fig. 11 is a flowchart showing a process performed in the second embodiment for determining a defocus amount prior to acquisition of a phase difference image. First, the culture container 50 in which fine beads that are markers are contained is provided on the stage 51 (step ST40). Then, the stage 51 is moved so that the observation region R of the imaging optical system 14 is set to the position of the scanning start point S shown in Fig. 6, and scanning according to the observation region R is started (step ST42).

Then, in the first observation region R, the captured image G0 for determining a defocus amount is acquired by the imaging unit 16 (step ST44), and the marker image detection section 31 detects a marker image from the captured image G0 (step ST46). Then, the discriminator 32 discriminates the defocus amount of the marker image included in the captured image G0 (step ST48), and the defocus amount determination section 33 determines the defocus amount in the observation region R (step ST50). Then, the operation controller 34 calculates a focus control amount on the basis of the determined defocus amount (step ST52), and stores the focus control amount in association with an X-Y coordinate position of the detection position of the culture container 50 (step ST54).

Then, in a case where the entire scanning is not terminated (step ST56; NO), the observation region R is moved in the X direction or the Y direction, and the acquisition of the captured image G0, the detection of the marker image, the discrimination of the defocus amount, the determination of the defocus amount, the calculation of the focus control amount, and the storage of the focus control amount that have been described above are repeatedly performed until the entire scanning is terminated (step ST44 to step ST54). Further, at a time point when the observation region R reaches the position of the scanning end point E shown in Fig. 9, the entire scanning is terminated (step ST56; YES).

In the second embodiment, in the acquisition of the phase difference image, the culture container 50 is scanned similarly to a case where the defocus amount is determined, and the operation controller 34 performs the autofocus control using the focus control amount stored in association with the X-Y coordinates of the culture container 50 corresponding to the observation region R in acquiring the phase difference image in each observation region R. Thus, the phase difference image is acquired while performing the focus control in each observation region R. In this case, it is necessary to scan the culture container 50 for storing the focus control amount in advance, but in a case where the same type of culture container 50 is used, in acquiring the phase difference image, in each observation region R, it is not necessary to stop the stage 51 once to perform the acquisition of the captured image G0, the detection of the marker image, the discrimination of the defocus amount, the determination of the defocus amount, the calculation of the focus control amount, the autofocus control, and the acquisition of the phase difference image. Thus, it is possible to continuously operate the observation region R on the culture container 50, and thus, it is possible to acquire the phase difference image at higher speed.

In addition, in the second embodiment, the operation controller 34 stores the focus control amount in each observation region R, but instead, the operation controller 34 may store the determined defocus amount. In this case, in acquiring the phase difference image in each observation region R, the focus control amount is calculated on the basis of the stored defocus amount, and the imaging of the observation region R and the acquisition of the phase difference image are performed.

By the way, both an image defocused in the positive direction and an image defocused in the negative direction are used as the teacher marker images used in the learning of the discriminator 32. However, in a case where the image defocused in the positive direction and the image defocused in the negative direction are similar to each other, even though the discriminator 32 that has performed learning using such teacher marker images is used, it may be difficult to discriminate whether the defocus amount is a positive defocus amount or a negative defocus amount.

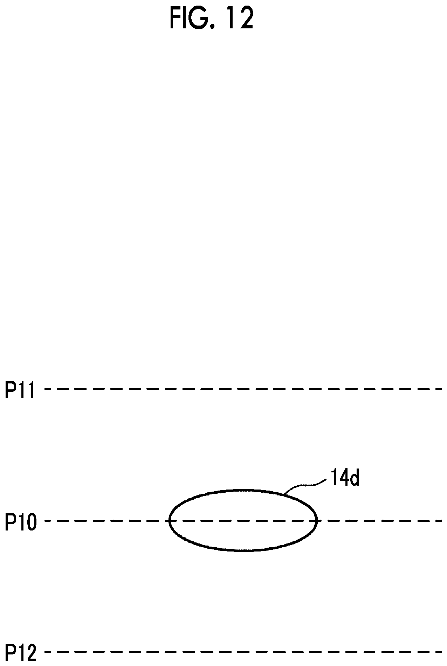

However, in the present embodiment, even if the positive and negative defocus amounts are mistakenly discriminated, it is possible to perform the autofocus control at high speed. Fig. 12 is a diagram for illustrating the autofocus control. Fig. 12 shows an autofocus control in a case where the imaging lens 14d is moved in the Z direction. As shown in Fig. 12, it is assumed that the defocus amount in a case where the imaging lens 14d is at a position P10 is determined to be + α. In this case, in a case where an actual defocus amount is positive (that is, a state where the focus is distant with reference to an observation target), the imaging lens 14d may be moved in a direction away from the observation target, for example, may be moved to a position P11 to be focused on the observation target. However, in a case where the focus is actually close with reference to the observation target and the defocus amount is -α, if the imaging lens 14d is moved to the position P11, the focus is further lost.

In this case, at a time point when the imaging lens 14d is moved to the position P11, the captured image G0 for determining the defocus amount is acquired again, and the defocus amount is determined. Then, in a case where the determined defocus amount is not 0, since the positive and negative of the defocus amount are incorrect, the operation controller 34 determines the focus control amount to move the imaging lens 14d in a direction closer to the observation target, for example, from the position P11 to a position P12.

Here, in a case where the autofocus control is performed by determining the contrast of an image as in the related art, it is necessary to repeat the acquisition of the captured image G0 and the determination of the focus control amount until the observation target is focused. On the other hand, in the present embodiment, even if the positive and negative focus control amount are erroneously discriminated, it is possible to determine an accurate focus control amount by only performing the operation of determining the defocus amount once again. Accordingly, in this embodiment, even if the positive and negative focus control amounts are erroneously discriminated, it is possible to perform the autofocus control at high speed.

In a case where the image defocused in the positive direction and the image defocused in the negative direction are similar to each other, the discriminator 32may perform learning using only one of the image defocused in the positive direction and the image defocused in the negative direction as the teacher marker image. For example, in a case where the discriminator 32 performs learning using only the image defocused in the positive direction as the teacher marker image, the defocus amount to be discriminated has a positive value. In this case, in a case where the actual defocus amount is negative, as shown in Fig. 12, in a case where the imaging lens 14d is moved to the position P11 as in the case where the defocus amount is positive, the focus is further lost.

In this case, at a time point when the imaging lens 14d is moved to the position P11, the captured image G0 for determining the defocus amount is acquired again, and the defocus amount is determined. Then, in a case where the determined defocus amount is not 0, it is determined that the defocus amount is actually negative, and the operation controller 34 determines the focus control amount to move the imaging lens 14d from the position P11 to P12. Thus, as in the case where the positive and negative of the defocus amount are mistaken, it is possible to determine an accurate focus control amount only by performing the operation of determining the defocus amount once again. Accordingly, even in a case where the discriminator 32 performs learning using only one of the image defocused in the positive direction and the image defocused in the negative direction as the teacher marker image, it is possible to perform the autofocus control at high speed.

In each of the above embodiments, the marker image of which the defocus amount is known is used as the teacher marker image for the learning of the discriminator 32, but the invention is not limited to thereto. For example, a marker image of which the defocus amount is not clear may be used as the teacher marker image. In this case, for the marker image of the defocus amount is not clear, the learning section 35 performs the learning of the discriminator 32 so as to discriminate that the defocus amount is not clear. As the marker image of which the defocus amount is not clear, a marker image of which the defocus amount is erroneously discriminated as a result of being input to the discriminator 32 may be used. Accordingly, the learning section 35 first performs the learning for the discriminator 32 so as not to discriminate that the defocus amount is not clear. Then, at a stage where the learning has progressed to some extent, in a case where the defocus amount is discriminated by the discriminator 32, the marker image of which the defocus amount is erroneously discriminated is determined as the marker image of which the defocus amount is not clear. Then, by using such a marker image again, the learning section 35 performs the learning of the discriminator 32 so as to discriminate that the defocus amount is not clear. Thus, it is possible to generate the discriminator 32 capable of discriminating that the defocus amount is not clear. Accordingly, it is possible to reduce a possibility that a wrong discrimination result of the defocus amount is acquired.

In the above-described embodiments, the operation section 15 performs the autofocus control by the first to seventh operation sections 15A to 15G, but the autofocus control may be performed using any one or a plurality of the first to seventh operation sections 15A to 15G. Further, any one or a plurality of the first to seventh operation sections 15A to 15G may be provided.

Further, in the above-described embodiments, the focal length changing optical system 70 is disposed between the imaging optical system 14 and the imaging unit 16, but instead, the focal length changing optical system 70 may be disposed between the imaging optical system 14 and the stage 51.

Further, in the above-described embodiments, the culture container 50 is moved in the optical axis direction by moving the stage 51 in the optical axis direction using the fourth operation section 15D. However, instead of moving the stage 51 in the optical axis direction, a mechanism for moving the culture container 50 in the optical axis direction may be provided, and only the culture container 50 may be moved in the optical axis direction.

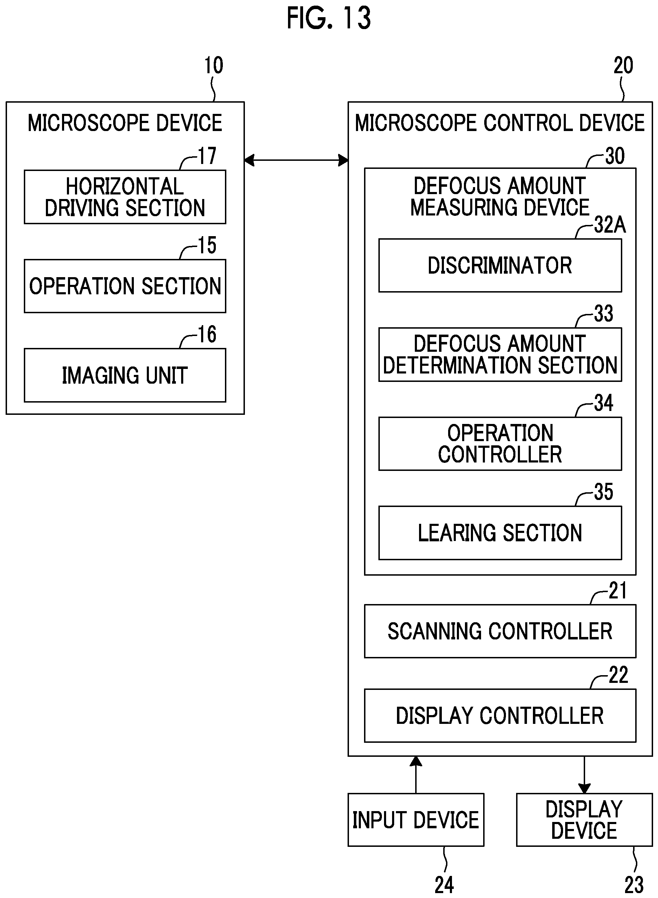

In the above-described embodiments, the discriminator 32 discriminates the defocus amount of the marker image detected from the captured image G0 by the marker image detection section 31. However, the presence or absence of the marker image in the captured image G0 may be discriminated by only a discriminator, and the defocus amount of the marker image may be discriminated in a case where the marker image is included. Hereinafter, this configuration will be described as a third embodiment. Fig. 13 is a block diagram showing a configuration of a microscope observation system according to the third embodiment. In Fig. 13, the same components as those in Fig.5 are designated by the same reference numerals, and detailed description thereof will not be repeated. As shown in Fig. 13, the third embodiment is different from the first embodiment in that, in the microscope control device 20, the marker image detection section 31 is not provided and a discriminator 32A is provided instead of the discriminator 32.

In the third embodiment, the discriminator 32A discriminates the presence or absence of a marker image in a captured image G0, and discriminates a defocus amount of the marker image in a case where the marker image is included in the captured image G0. The learning section 35 performs learning of the discriminator 32A using a teacher image that does not include the marker image in addition to a teacher marker image of which the defocus amount is known. As the teacher image that does not include the marker image, the above-described marker image of which the defocus amount is erroneously discriminated may be used.

In the third embodiment, since the discriminator 32A that performs learning in this way is provided, even in a case where the marker image detection section 31 is not provided, it is possible to measure the defocus amount of the marker image included in the captured image G0.

Further, in the above-described embodiments, the defocus amount measuring device according to the present disclosure is applied to the phase difference microscope, but the present disclosure is not limited to the phase difference microscope, and may be applied to a different microscope such as a differential interference microscope, a bright field microscope.

Hereinafter, effects of the present embodiments will be described.

By discriminating a defocus amount for each of a plurality of marker images included in a captured image and determining a statistical value of the plurality of defocus amounts as a defocus amount of the captured image, it is possible to absorb variation in discrimination results of the discriminator, to thereby accurately determine the defocus amount.

By discriminating that the defocus amount is not clear in the discriminator, it is possible to reduce a possibility that an incorrect defocus amount discrimination result is acquired.

By setting a marker to a fine cell structure, it is not necessary to prepare a special marker, and thus, it is possible to determine a defocus amount while imaging cells.

By imaging a container that includes a marker and contains an observation target to acquire a captured image and focusing the image of the observation target in the container on the imaging unit on the basis of a defocus amount, it is possible to determine the defocus amount at high speed, and thus, it is possible to perform a focus operation at high speed.

By scanning an observation region in a container provided on a stage where the container that contains an observation target is placed, performing imaging of each observation region in the container, and focusing the image of the observation target in the container on the imaging unit on the basis of the defocus amount in each observation region, it is possible to perform tiling imaging at high speed.

### Explanation of References

- 10:: Microscope device
- 11:: White light source
- 12:: Condenser lens
- 13:: Slit plate
- 14:: Imaging optical system
- 14a:: Phase difference lens
- 14b:: Objective lens
- 14c:: Phase plate
- 14d:: Imaging lens
- 15:: Operation section
- 15A:: First operation section
- 15B:: Second operation section
- 15C:: Third operation section
- 15D:: Fourth operation section
- 15E:: Fifth operation section
- 15F:: Sixth operation section
- 15G:: Seventh operation section
- 16:: Imaging unit
- 17:: Horizontal driving section
- 20:: Microscope control device
- 21:: Scanning controller
- 22:: Display controller
- 23:: Display device
- 24:: Input device
- 30:: Defocus amount measuring device
- 31:: Marker image detection section
- 32, 32A:: Discriminator
- 33:: Defocus amount determination section
- 34:: Operation controller
- 35:: Learning section
- 50:: Culture container
- 51:: Stage
- 51a:: Opening
- 70:: Focal length changing optical system
- 71:: First wedge prism
- 72:: Second wedge prism
- J:: Scanning position by observation region
- S:: Scanning start point
- E:: Scanning end point
- L:: Illumination light
- R:: Observation region
- W:: Well

## Claims

1. A defocus amount measuring device comprising:
a marker image detection section that detects a marker image from a captured image acquired by imaging a marker that is a measurement target of a defocus amount; and
a discriminator that performs learning using feature amounts related to a plurality of teacher marker images captured with various defocus amounts and discriminates a defocus amount of an input marker image.

2. The defocus amount measuring device according to claim 1,
wherein the discriminator discriminates the defocus amount for each of a plurality of the marker images included in the captured image, and
wherein the defocus amount measuring device further comprises:
a defocus amount determination section that determines a statistical value of a plurality of the defocus amounts as the defocus amount of the captured image.

3. The defocus amount measuring device according to claim 1 or 2,
wherein the discriminator discriminates that the defocus amount is not clear.

4. The defocus amount measuring device according to any one of claims 1 to 3,
wherein the discriminator is configured by a neural network.

5. The defocus amount measuring device according to any one of claims 1 to 4,
wherein the discriminator learns a co-occurrence matrix related to the plurality of teacher marker images as the feature amount.

6. The defocus amount measuring device according to any one of claims 1 to 5,
wherein the marker has a fine cell structure.

7. The defocus amount measuring device according to any one of claims 1 to 6,
wherein the captured image includes the marker and is acquired by imaging a container in which an observation target is contained, by an imaging unit, and
wherein the defocus amount measuring device further comprises:
a controller that performs a control for focusing an image of the observation target in the container on the imaging unit on the basis of the defocus amount.

8. The defocus amount measuring device according to claim 7, further comprising:
a stage on which the container in which the observation target is contained is placed,
wherein the captured image is acquired by scanning an observation region in the container placed on the stage and performing imaging of each observation region in the container, and
wherein the controller performs the control for focusing the image of the observation target in the container on the imaging unit on the basis of the defocus amount, in each observation region.

9. A defocus amount measuring method comprising:
detecting a marker image from a captured image acquired by imaging a marker that is a measurement target of a defocus amount; and
discriminating, using a discriminator that performs learning using feature amounts related to a plurality of teacher marker images captured with various defocus amounts and that discriminates a defocus amount of an input marker image, the defocus amount of the input marker image.

10. A computer-readable storage medium that stores a defocus amount measuring program causing a computer to execute:
a process of detecting a marker image from a captured image acquired by imaging a marker that is a measurement target of a defocus amount; and
a process of discriminating, using a discriminator that performs learning using feature amounts related to a plurality of teacher marker images captured with various defocus amounts and that discriminates a defocus amount of an input marker image, the defocus amount of the input marker image.

11. A discriminator that performs learning using feature amounts related to a plurality of teacher marker images captured with various defocus amounts and discriminates a defocus amount of an input marker image.

12. A defocus amount measuring device comprising:
a discriminator that performs learning using feature amounts related to a plurality of teacher marker images captured with various defocus amounts and in a case where a captured image acquired by imaging a marker that is a measurement target of a defocus amount is input, discriminates a presence or absence of a marker image in the captured image and a defocus amount of the marker image in a case where the marker image is included in the captured image.

13. A discriminator that performs learning using feature amounts related to a plurality of teacher marker images captured with various defocus amounts and in a case where a captured image acquired by imaging a marker that is a measurement target of a defocus amount is input, discriminates a presence or absence of a marker image in the captured image and a defocus amount of the marker image in a case where the marker image is included in the captured image.
